# EUROPEAN PATENT SPECIFICATION

(11) **EP 3 162 363 B1**
(45) Date of publication and mention of the grant of the patent: **08.01.2020**
(21) Application number: 15816054.9
(22) Date of filing: 30.06.2015
(51) Int. Cl.: A61K 9/20, A61K 9/48, A61K 47/32, A61K 47/30, A61K 45/06

(54) **COMPOSITE PREPARATION COMPRISING ACTIVE INGREDIENT-CONTAINING FILM COATING LAYER**
ZUSAMMENGESETZTE ZUBEREITUNG MIT WIRKSTOFFHALTIGER FILMBESCHICHTUNG
PRÉPARATION COMPOSITE COMPRENANT UNE COUCHE DE REVÊTEMENT PAR FILM CONTENANT UN PRINCIPE ACTIF

(30) Priority: 30.06.2014 KR 20140081223
(43) Date of publication of application: 03.05.2017
(73) Proprietor: Hanmi Pharm. Co., Ltd., Hwaseong-si, Gyeonggi-do 18536 (KR)
(72) Inventor: KIM, Hyung Seo, Hwaseong-si Gyeonggi-do 445-925 (KR); CHO, Jung Hyun, Hwaseong-si Gyeonggi-do 445-330 (KR); CHOI, Young Keun, Suwon-si Gyeonggi-do 440-824 (KR); KIM, Kyeong Soo, Suwon-si Gyeonggi-do 440-710 (KR); KIM, Jin Cheul, Seoul 150-754 (KR); KIM, Yong Il, Suwon-si Gyeonggi-do 440-730 (KR); PARK, Jae Hyun, Suwon-si Gyeonggi-do 443-737 (KR); WOO, Jong Soo, Suwon-si Gyeonggi-do 440-710 (KR)
(74) Representative: Marks & Clerk (Luxembourg) LLP
(86) International application number: PCT/KR2015/006743
(87) International publication number: WO 2016/003181

(56) References cited:
- WO-A1-2006/108519
- WO-A1-2014/017833
- WO-A1-2014/182003
- KR-A- 20080 007 252
- KR-A- 20140 013 436
- US-A1- 2013 171 199
- US-A1- 2013 171 199
- US-A1- 2014 010 872
- 'Technical Information, Kollicoat Protect, 03_040903e-08' BASF, PHARMA INGREDIENTS & SERVICES February 2012, pages 1 - 10, XP055380857 Retrieved from the Internet: <URL:http://www.pharma-ingredients.basf.com /Statements/Techcical%20 Informations/EN/Pharma%20Solutions/03_04090 3e_Kollicoat%20Protect.pdf>

## Description

### TECHNICAL FIELD

The present disclosure relates to a composite preparation including an active ingredient-containing film coating layer and a preparation method thereof, and more particularly, to a composite preparation including an active ingredient-containing film coating layer which may have a stable shape and durability against common external impacts and a high dissolution rate of the active ingredient, and a preparation method of the composite preparation.

### BACKGROUND ART

The prostate gland of a healthy young man is about the size of a walnut, and gradually grows with age. Prostatic hypertrophy is a disease of severe enlargement of the prostate gland in which the urethra passing through the prostate is squeezed, leading to various symptoms.

Although not clearly identified yet, the cause of prostatic hypertrophy is known to be various complex factors, as in other chronic diseases. A currently recognized cause of prostatic hypertrophy is aging of the normal testicles. The prostate gland is an androgenic hormone (male sex hormone)-dependent organ which needs continuous supply of the androgenic hormone to grow and function normally. The prostate gland may shrink if the androgenic hormone is not produced any longer due to castration.

Symptoms of prostatic hypertrophy include lower urinary tract symptoms (LUTS), including symptoms caused by bladder storage disorders, such as frequent urination of eight times or more a day, nocturia, urgent urination accompanied by a strong, sudden sensation to urinate and compelling urge to urinate, urgent urinary incontinence, and painful urination (dysuria); and symptoms caused by bladder discharge disorders, such as retarded urination (in which the patient must wait for urination to begin), interrupted urination (with an intermittent stream of urine), stress urination (in which the patient must apply pressure for urination to begin), weak urinary stream, a sensation of incomplete emptying, and urinary retention.

5-α-reductase inhibitors are drugs typically used to treat prostatic hypertrophy, and may be used alone, but are also known to be used together with tamsulosin or a phosphodiesterase-5 inhibitor (for example, tadalafil, vardenafil, or udenafil) for more effective treatment of prostatic hypertrophy.

5-α-reductase inhibitors may inhibit the conversion of testosterone to dihydrotestosterone which further enhances the enlargement of the prostate gland, and thus may block the enlargement of the prostate and relieve the urinary tract from physical pressure.

Tamsulosin is a drug which selectively blocks α-adrenoceptors in the urogenital organs, and is known to improve urination rates by relaxing the prostate and the smooth muscle surrounding the bladder, so as to alleviate the symptoms of benign prostatic hypertrophy.

Tadalafil (Cialis, Lilly ICOS) and vardenafiln (Levitra, GSK), as phosphodiesterase-5 inhibitors, were originally developed as drugs for the treatment of impotency. However, these drugs are increasingly being used for more indications, such as prostatic hypertrophy and overactive bladder (Euro Urol 2008; 1236-44; J Uro 2007;1401-7).

Drug combinations, such as that of a 5-α-reductase inhibitor with tamsulosin, or that of a 5-α-reductase inhibitor with a phosphodiesterase-5 inhibitor, have advantages in terms of efficacy enhancement due to the different action mechanisms of the two active ingredients administered in combination (EP 1 501 517; BJU Int. 2006 Apr. 97 Suppl 2:39-43; discussion 44-5).

However, in preparing a composite preparation including two or more active ingredients, such as the drug combinations as described above, problems relating to the stability of the active ingredients may arise due to interaction between the two or more different active ingredients. To ensure stability of such a composite preparation, a composite preparation has been developed which includes an active ingredient-containing core and a film coating layer containing another active ingredient, in which the two active ingredients are separated into different layers (EP 1 830 820; US 6,682,759). US 2013/0171199 discloses an oral dispersible tablet comprising sustained release tamsulosin pellets coated with a drug dispersion comprising finasteride.

In such a composite preparation including an active ingredient-containing film coating layer, if the film coating layer has an insufficient tensile strength, the film coating layer may become separated or broken by common impacts during storage of the product, such that it may be difficult to ensure sufficient efficacy of the composite preparation and maintain merchantable product quality due to changes in appearance and other properties, eventually leading to the inability to commercialize the preparation as a product. For such a composite preparation to be administered orally, a high dissolution rate of an active ingredient contained in the film coating layer is required to ensure sufficient bioavailability and rapid efficacy.

### DETAILED DESCRIPTION OF THE INVENTION

### TECHNICAL PROBLEM

The present disclosure provides a composite preparation including an active ingredient-containing film coating layer, which may have a stable shape and durability against common external impacts and a high dissolution rate of the active ingredient.

The present disclosure provides a method of preparing the composite preparation including the active ingredient-containing film coating layer.

### TECHNICAL SOLUTION

According to an aspect of the present invention, there is provided a composite preparation including: a core containing a first active ingredient; and a film coating layer containing a second active ingredient, wherein the film coating layer comprises a polyvinyl alcohol-polyethylene glycol graft copolymer and polyvinyl alcohol, wherein a weight ratio of the polyvinyl alcohol-polyethylene glycol graft copolymer to the polyvinyl alcohol is 7:3 to 4:6, and wherein the second active ingredient comprises a 5-α-reductase inhibitor selected from the group consisting of finasteride, dutasteride and any combination thereof.

According to another aspect of the present invention, there is provided a method of preparing the above-described composite preparation, the method including: preparing the core containing the first active ingredient; preparing a second active ingredient-containing coating solution in which the second active ingredient, a polyvinyl alcohol-polyethylene glycol graft copolymer, and polyvinyl alcohol are dissolved in a solvent; and coating the core with the second active ingredient-containing coating solution.

### ADVANTAGEOUS EFFECTS

According to the one or more embodiments of the present disclosure, a composite preparation may include an active ingredient-containing film coating layer which has strong tensile strength durable against common external impacts, and ensures a high dissolution rate of the active ingredient contained in the film coating layer. Thus, the composite preparation may have high merchantable product quality since it undergoes no change in appearance and properties as a result of external impacts, and may ensure sufficient bioavailability and rapid action without loss of efficacy. The composite preparation containing two or more different active ingredients may be used as an effective composite formulation with good merchantable product quality, and may improve patient compliance for the combined administration of drugs. Furthermore, due to the inclusion of the film coating layer in the composite preparation, the composite preparation may have a remarkably reduced volume, and thus may be in the form of remarkably reduced size of a tablet or capsule compared to a composite formulation made by simply combining the composition of each of the single preparation, increasing the convenience to the patient when taking the medication.

### BRIEF DESCRIPTION OF THE DRAWINGS

FIG. 1 is a schematic view illustrating a composite preparation (a) including a second active ingredient-containing film coating layer coated on a surface of a first active ingredient-containing tablet core, and a composite preparation (b) including a second active ingredient-containing film coating layer coated on a surface of a first active ingredient-containing hard capsule core;
FIG. 2 is a photographic image of composite preparations, one of which is evaluated as an acceptable product (a) and the other as a defective product (b);
FIG. 3 is a graph illustrating defect rates of composite preparations of Comparative Examples 1 to 8, evaluated upon unpacking PTP packages thereof;
FIG. 4 is a graph illustrating defect rates of composite preparations of Examples 1 to 10, evaluated upon unpacking PTP packages thereof;
FIG. 5 is a graph illustrating defect rates with respect to coating materials of composite preparations of Comparative Examples 1 to 4, evaluated upon unpacking PTP packages thereof after storage for one week at about 60°C and at a relative humidity (RH) of 0%;
FIG. 6 is a graph illustrating dissolution rates of finasteride from the composite preparations of Comparative Examples 1 to 4;
FIG. 7 is a graph illustrating dissolution rates of finasteride from the composite preparations of Examples 2 to 5; and
FIG. 8 is a graph illustrating defect rates with respect to different core types of composite preparations of Examples 3, 11 and 12, evaluated upon unpacking PTP packages thereof.

### MODE FOR INVENTION

Unless otherwise defined, all terms (including technical and scientific terms) used herein have the same meaning as commonly understood by one of ordinary skill in the art to which this invention belongs.

As a result of in-depth research into a coating material for a composite preparation including an active ingredient-containing film coating layer, the coating material ensuring a sufficient tensile strength of the active ingredient-containing film coating layer and a high dissolution rate of the active ingredient contained in the film coating layer, the inventors of the present disclosure found that when a combination of a polyvinyl alcohol-polyethylene glycol graft copolymer and polyvinyl alcohol is used as a material of the active ingredient-containing film coating layer, as compared when other film coating materials are used, the active ingredient-containing film coating layer may have a tensile strength strong enough not to be separated or broken during storage by common impacts, and a high dissolution rate of the active ingredient contained in the film coating layer.

An aspect of the present disclosure provides a composite preparation as defined in claim 1 including: a core containing a first active ingredient; and a film coating layer containing a second active ingredient, wherein the film coating layer includes a polyvinyl alcohol-polyethylene glycol graft copolymer and polyvinyl alcohol.

The core containing the first active ingredient may be any pharmaceutical formulation commonly used in the field of pharmaceuticals. For example, the core may be in the form of a tablet, a hard capsule, or a soft capsule. However, embodiments are not limited thereto. A filler part of the hard capsule or soft capsule may be in the form of any pharmaceutical formulation commonly used in the field of pharmaceuticals, for example, granules, pellets, powder, tablets, liquid, or any combinations thereof.

The core may be about 20 wt% to 99.5 wt% of a total weight of the composite preparation.

The film coating layer containing the second active ingredient may be on a surface of the core. The film coating layer comprises a combination of a polyvinyl alcohol-polyethylene glycol graft copolymer and polyvinyl alcohol as a coating material.

The inventors of the present invention prepared composite preparations using various coating materials by coating tablet or capsule cores containing a first active ingredient with a film coating layer including a second active ingredient and a different coating material, and conducted a detect test with respect to the different coating materials and a dissolution test of the second active ingredient with time. As a result, when a polyvinyl alcohol-polyethylene glycol graft copolymer, polyvinyl alcohol, povidone, or hypromellose was used alone as a coating material, a defect rate of the film coating layer of the composition preparation, resulting from such as separation from the core or breaking, was as high as 20 to 40%, and was less than 2% only when polyvinyl alcohol was used (see Test Examples 1 and 3). However, when polyvinyl alcohol was used as the coating material, the composition preparation had a low dissolution rate of the second active ingredient of less than 75% in 15 minutes, as evaluated using a Dissolution Test of the General Tests of the *Korean Pharmacopoeia* (see Test Example 4). Accordingly, it was difficult to obtain a film coating layer that ensures both a high tensile strength and a sufficiently high dissolution rate. On the other hand, when a combination of a polyvinyl alcohol-polyethylene glycol graft copolymer and polyvinyl alcohol was used, a defect rate of the film coating layer, resulting from factors such as separation from the core or breaking, was markedly reduced to less than 10% (see Test Examples 2 and 6) and the second active ingredient had a sufficiently high dissolution rate of about 75% or greater in 15 minutes (see Test Example 5), indicating remarkable results showing that both a low film coating defect rate and a high dissolution rate were achieved.

The polyvinyl alcohol-polyethylene glycol graft copolymer and polyvinyl alcohol are used in a weight ratio of 7:3 to 4:6, and in some embodiments, about 6:4.

The polyvinyl alcohol-polyethylene glycol graft copolymer may consist of about 65% to about 85% of a polyvinyl alcohol unit and about 15 to 35% of a polyethylene glycol unit, may contain about 0.01% to about 0.5% of colloid silica, and may have a weight average molecular weight of about 35,000 to about 55,000 Daltons. The polyvinyl alcohol-polyethylene glycol graft copolymer is commercially available, for example, as Kollicoat® IR (available from BASF), which consists of about 75% of polyvinyl alcohol unit and about 25% of polyethylene glycol unit, contains about 0.3% of colloid silica, and has a weight average molecular weight of about 45,000 Daltons.

The polyvinyl alcohol, which is a water soluble polymer, may have a molecular weight of about 20,000 to about 200,000 Daltons, wherein the larger the molecular weight becomes, the viscosity may become higher.

The film coating layer may be about 0.5 parts to about 80 parts by weight with respect to 100 parts by weight of the core.

In some embodiments, the polyvinyl alcohol-polyethylene glycol graft copolymer of the composite preparation may include about 65% to about 85% of the polyvinyl alcohol unit and about 15% to about 35% of the polyethylene glycol unit, may contain about 0.01 to about 0.5wt% of colloid silica, and may have a weight average molecular weight of about 35,000 to about 55,000 Daltons; the polyvinyl alcohol may have a molecular weight of about 20,000 to about 200,000 Daltons.

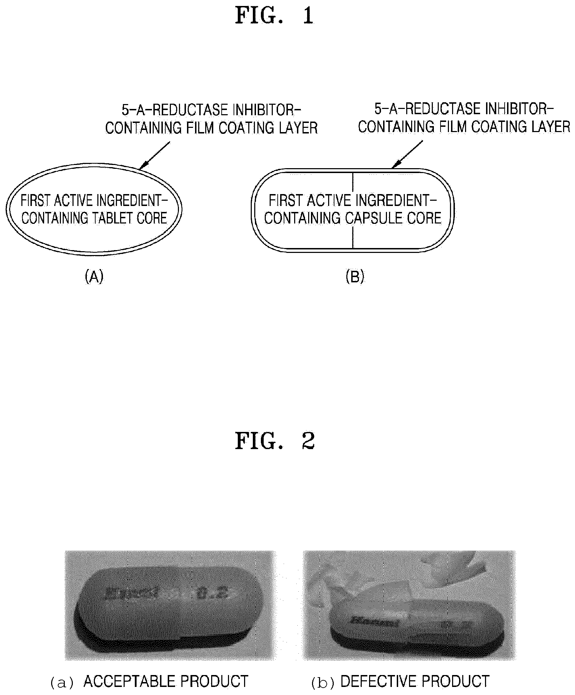

FIG. 1 is a schematic view illustrating composite preparations according to embodiments.

In FIG. 1, (a) is a schematic view illustrating a composite preparation in which a film coating layer containing a second active ingredient is coated on a surface of a tablet core including a first active ingredient; and (b) is a schematic view illustrating a composite preparation in which a film coating layer containing a second active ingredient is coated on a surface of a hard-capsule core containing a first active ingredient.

In some embodiments, the composite preparation may further include an inner coating layer that separates the core and the film coating layer from each other to more effectively prevent interaction between the active ingredients. The inner coating layer may be about 0.01 parts by weight to about 60 parts by weight with respect to 100 parts by weight of the core. For example, a film forming material (a film forming agent and/or coating agent) that may be used for the inner coating layer may be hydroxypropyl methyl cellulose, hydroxypropyl cellulose, hydroxyethyl cellulose, cellulose acetate phthalate, ethyl cellulose, methyl cellulose, polymethacrylate, polyethylene glycol, talc, titanium dioxide, or a mixture thereof. However, embodiments are not limited thereto. Any film forming materials commonly used to form solid formulations for oral or parenteral administration in the field of pharmaceuticals may be used.

In some embodiments, the composite preparation may further include an outer coating layer surrounding the active ingredient-containing film coating layer to further protect the composite preparation from external environments. The outer coating layer may be any coating layer that does not significantly affect a high tensile strength of the active ingredient-containing film coating layer and a high dissolution rate of the second active ingredient for which the composite preparation was designed. Such an outer coating layer may be obtained by moisture-proofing coating or polishing coating. An appropriate material may be chosen depending on a type of coating by one of ordinary skill in the art based on the known knowledge in the art.

The outer coating layer may be about 0.01 parts by weight to about 60 parts by weight with respect to 100 parts by weight of the core.

In some embodiments, the composite preparation may further include, in addition to the above-described ingredients, a pharmaceutically acceptable additive in the core and the active ingredient-containing film coating layer. The additive may be selected from the group consisting of a diluent, a disintegrant, a binder, a stabilizer, a lubricant, and any combinations thereof.

The diluent may be selected from the group consisting of microcrystalline cellulose, lactose, Rudy press, mannitol, calcium dihydrogen phosphate, starch, low-substituted hydroxypropyl cellulose, and any combinations thereof. However, embodiments are not limited thereto.

The disintegrant may be selected from the group consisting of crospovidone, sodium starch glycolate, croscarmellose sodium, low-substituted hydroxypropyl cellulose, starch, alginic acid, sodium alginate, and any combinations thereof. However, embodiments are not limited thereto.

The binder may be selected from the group consisting of hydroxypropyl cellulose, hydroxypropyl methyl cellulose, polyvinylpyrrolidone, copovidone, macrogol, light anhydrous silicic acid, silicate derivatives such as synthetic aluminum silicate, calcium silicate, and magnesium metasilicate aluminate, phosphates such as calcium hydrogen phosphate, carbonates such as calcium carbonate, and any combinations thereof. However, embodiments are not limited thereto.

The stabilizer may be selected from the group consisting of magnesium carbonate, sodium bicarbonate, sodium carbonate, calcium carbonate, and any combinations thereof. However, embodiments are not limited thereto.

The lubricant may be selected from the group consisting of stearic acid, metal salts of stearic acid such as calcium stearate or magnesium stearate, talc, colloid silica, sucrose fatty acid ester, hydrogenated vegetable oil, high-melting point wax, glyceryl fatty acid esters, glycerol dibehenate, and any combinations thereof. However, embodiments are not limited thereto.

As used herein, the term "active ingredient" means not only any pharmacological active agent for treatment purpose but also any reagent or any medical agent that may be administered into the human body for diagnostic or preventive purposes. In a broader sense, the "active ingredient" may be any material, ingredient, or healthy functional food in a processed form thereof that may be taken for health care purposes to carry out nutrient regulatory functions or physiological functions affecting the body structure and functions.

As used herein, the terms "first active ingredient" and "second active ingredient" are used to distinguish the at least two active ingredients of the composite preparation from one another, wherein, for ease of description, the active ingredient contained in the core of the composite preparation is referred to as a first active ingredient, and the active ingredient contained in the film coating layer is referred to as a second active ingredient.

The first active ingredient and the second active ingredient may be tamsulosin and a 5-α-reductase inhibitor as defined above, or a phosphodiesterase-5 inhibitor and a 5-α-reductase inhibitor as defined above. Each of the first active ingredient and the second active ingredient may include a single ingredient or a plurality of ingredients if needed.

In some embodiments, the composite preparation may be an oral dosage form.

In some embodiments, the first active ingredient may be any active ingredients which need to be administered in combination with a 5-α-reductase inhibitor. For example, the first active ingredient may include tamsulosin or a phosphodiesterase-5 inhibitor (for example, tadalafil, vardenafil, udenafil, or sildenafil).

A combination of a 5-α-reductase inhibitor and tamsulosin, and a combination of a 5-α-reductase inhibitor and a phosphodiesterase-5 inhibitor are also known to be effective in treating prostatic hypertrophy, and are also known to be advantageous in terms of efficacy enhancement due to the different mechanisms of the individual active ingredients(EP 1 501 517; BJU Int. 2006 Apr. 97 Suppl 2:39-43; Discussion 44-5). Thus, when including tamsulosin or a phosphodiesterase-5 inhibitor as the first active ingredient and a 5-α-reductase inhibitor as the second active ingredient, the composite preparation according to any of the embodiments may be used as an effective prostate treatment formulation.

In some embodiments, the first active ingredient may be tamsulosin or a pharmaceutically acceptable salt thereof, and the second active ingredient comprises a 5-α-reductase inhibitor.

The 5-α-reductase inhibitor is selected from the group consisting of finasteride, dutasteride, and any combinations thereof. A dissolution rate in 15 minutes of the 5-α-reductase inhibitor in the composite preparation may be about 75% or greater, as evaluated using a Dissolution Test according to the General Tests of the *Korean Pharmacopoeia.*

In some embodiments, in consideration of a known daily dose, the composite preparation may include about 0.1 mg to about 0.8 mg, and in some other embodiments, about 0.2 mg to about 0.6 mg of tamsulosin or a pharmaceutically acceptable salt thereof, expressed in terms of the amount of free base, as the first active ingredient, and about 1 mg to about 10 mg of finasteride as the second active ingredient.

In some embodiments, in consideration of a known daily dose, the composite preparation may include about 0.1 mg to about 0.8 mg, and in some other embodiments, about 0.2 mg to about 0.6 mg of tamsulosin or a pharmaceutically acceptable salt thereof, expressed in terms of the amount of free base, as the first active ingredient, and about 0.2 mg to about 0.6 mg of dutasteride as the second active ingredient.

In some embodiments, the first active ingredient may be a phosphodiesterase-5 inhibitor. The phosphodiesterase-5 inhibitor may be tadalafil, vardenafil, udenafil, sildenafil, or any combinations thereof. For example, the phosphodiesterase-5 inhibitor may be tadalafil. The tadalafil may be used as a tadalafil free base or a pharmaceutically acceptable salt thereof, for example, hydrobromide, phosphate, sulfate, hydrochloride, maleate, fumarate, lactate, tartrate, citrate, besylate, camsylate, or gluconate. For example, the tadalafil may be a tadalafil free base. However, embodiments are not limited thereto.

The 5-α-reductase inhibitor is selected from the group consisting of finasteride, dutasteride, and any combinations thereof. A dissolution rate in 15 minutes of the 5-α-reductase inhibitor of the composite preparation may be about 75% or greater, as evaluated using a Dissolution Test according to the General Tests of the *Korean Pharmacopoeia.*

In some embodiments, in consideration of a known daily dose, the composite preparation may include about 5 mg to about 20 mg, and in some other embodiments, about 5 mg to about 10 mg of tadalafil or a pharmaceutically acceptable salt thereof, expressed in terms of the amount of free base, as the first active ingredient, and about 1 mg to about 10 mg of finasteride as the second active ingredient.

In some embodiments, in consideration of a known daily dose, the composite preparation may include about 5 mg to about 20 mg, and in some other embodiments, about 5 mg to about 10 mg of tadalafil or a pharmaceutically acceptable salt thereof, expressed in terms of the amount of free base, as the first active ingredient, and about 0.2 mg to about 0.6 mg of dutasteride as the second active ingredient.

In some embodiments, the composite preparation may include tamsulosin or a pharmaceutically acceptable salt thereof as the first active ingredient, and a 5-α-reductase inhibitor as defined above as the second active ingredient; the polyvinyl alcohol-polyethylene glycol graft copolymer of the film coating layer may consist of about 65% to about 85% of a polyvinyl alcohol unit and about 15% to about 35% of a polyethylene glycol unit, may include about 0.01wt% to about 0.5wt% of colloid silica, and may have a weight average molecular weight of about 35,000 Daltons to about 55,000 Daltons; and the polyvinyl alcohol of the film coating layer may have a molecular weight of about 20,000 Daltons to about 200,000 Daltons, and a weight ratio of the polyvinyl alcohol-polyethylene glycol graft copolymer to the polyvinyl alcohol is 7:3 to 4:6.

A composite preparation according to an embodiment including tamsulosin, a pharmaceutically acceptable salt thereof, or a phosphodiesterase-5 inhibitor as the first active ingredient and a 5-α-reductase inhibitor as the second active ingredient may have an improvement in shape and durability due to enhanced tensile strength of the film coating layer, and ensure rapid release of the 5-α-reductase inhibitor. Furthermore, due to the inclusion of the two active ingredients effective for treating prostatic hypertrophy with different mechanisms, in a single formulation, the composite preparation may provide a synergistic effect in treating and alleviating prostatic hypertrophy, and may also increase patient medication compliance.

An aspect of the present invention provides a method of preparing a composite preparation according to any of the above-described embodiments, the method including: preparing a core containing the first active ingredient; preparing a second active ingredient-containing coating solution in which the second active ingredient, a polyvinyl alcohol-polyethylene glycol graft copolymer, and polyvinyl alcohol are dissolved in a solvent; and coating the core with the coating solution.

The above-described details of the composite preparations according to all the aspects described above may apply to the preparation methods thereof according to embodiments.

In preparing the core, the core may be any core capable of being used as a core in the field of pharmaceutics, in the form of, for example, a tablet, a hard capsule, or a soft capsule. However, embodiments are not limited thereto. In preparing the core, any commercially available formulation which may be used as a core may be used, or the core may be directly prepared for use in the invention. The core may be prepared using a known technology in the field of pharmaceutics, chosen according to a type of the core, by one of one of ordinary skill in the art.

In preparing the second active ingredient-containing coating solution, the solvent may be any pharmaceutically available solvent which may dissolve the second active ingredient, polyvinyl alcohol-polyethylene glycol graft copolymer, and polyvinyl alcohol. For example, the solvent may be water, ethanol, methanol, chloroform, dimethyl sulfoxide (DMSO), or any combination solution thereof. In some embodiments, the solvent may be water, ethanol, or any combination solution thereof. However, embodiments are not limited thereto.

The coating of the core may be performed by any film coating method available used in the field of pharmaceuticals, for example, a pan-coating method, a fluidized-bed coating, or a press-coating method. However, embodiments are not limited thereto.

When a 5-α-reductase inhibitor, especially finasteride known to be teratogenic, is used as the second active ingredient, a separate independent production line is required to prevent finasteride from being mixed with other medical products during preparation of a finasteride-containing formulation by a pharmaceutical company. For example, in preparing a solid formulation containing finasteride, all processes, including mixing, granulating, tableting, and coating, must be performed in a segregated and exclusive area. However, a method of preparing a composite preparation according to an embodiment involves applying finasteride with a finasteride-containing coating solution, so that the existing drug preparation facilities may be used just by additionally introducing an exclusive coating facility for finasteride-containing coating, and thus preparing a finasteride formulation may be relatively convenient. Therefore, when using a method of preparing a composite preparation, according to an embodiment, a 5-α-reductase inhibitor-containing composite preparation may be prepared more economically.

### MODE OF THE INVENTION

One or more embodiments of the present invention will now be described in detail with reference to the following examples. However, these examples are only for illustrative purposes and are not intended to limit the scope of the one or more embodiments of the present invention.

### Examples 1-10: Preparation of composite preparations including tamsulosin hydrochloride capsule core coated with 5-α-reductase inhibitor-containing film coating layer (1)

Coating solutions were prepared by mixing coating materials having the different compositions (Examples 1 to 10) presented in Table 1 with an ethanol-water mixture solution (ethanol:water = 1:1 (v/v)), and then coated on tamsulosin hydrochloride-containing Tamsulosin® capsules (available from Hanmi Pharmaceutical Co., Ltd., Republic of Korea) by using a pan coater (SFC-30, Sejong Co., Ltd.). The prepared capsules were dried at about 35°C for about 30 minutes, thereby preparing composite preparations including a tamsulosin capsule core coated with a 5-α-reductase inhibitor-containing film coating layer.

### Comparative Examples 1 to 8: Preparation of composite preparations including tamsulosin hydrochloride capsule core coated with 5-α-reductase inhibitor-containing film coating layer (2)

Composite preparations of Comparative Examples 1 to 8 including a tamsulosin capsule core coated with a 5-α-reductase inhibitor-containing film coating layer were prepared in the same manners as in Examples 1 to 10, except that the coating solutions were prepared according to the compositions for Comparative Examples 1 to 8 presented in Table 1.

**[Table 1]**

| | MAIN INGREDIENT (mg) | | COATING MATERIAL (mg) | | | | RATIO OF KOLLICOAT® IR TO POLYVINYL ALCOHOL |
|---|---|---|---|---|---|---|---|
| | FINASTERIDE | DUTASTERIDE | KOLLICOAT® IR | POLYVINYL ALCOHOL | POVIDONE | HYPROMELLOSE | |
| Comparative Example 1 | 5 | - | 40. 0 | | | | - |
| Camparative Example 2 | 5 | - | | 40.0 | | | - |
| Comparative Example 3 | 5 | - | | | 40.0 | | - |
| Comparative Example 4 | 5 | - | | | | 40.0 | - |
| Example 1 | 5 | - | 32.0 | 8.0 | | | 8 : 2 |
| Example 2 | 5 | - | 28.0 | 12.0 | | | 7 : 3 |
| Example 3 | 5 | - | 24.0 | 16.0 | | | 6 : 4 |
| Example 4 | 5 | - | 20.0 | 20.0 | | | 5 : 5 |
| Example 5 | 5 | - | 16.0 | 24.0 | | | 4 : 6 |
| Comparative Example 5 | - | 0.5 | 40.0 | | | | - |
| Comparative Example 6 | - | 0.5 | | 40.0 | | | - |
| Comparative Example 7 | - | 0.5 | | | 40.0 | | - |
| Comparative Example B | - | 0.5 | | | | 40.0 | - |
| Example 6 | - | 0.5 | 32.0 | 8.0 | | | 8 : 2 |
| Example 7 | - | 0.5 | 28.0 | 12.0 | | | 7 : 3 |
| Example 8 | - | 0.5 | 24.0 | 16.0 | | | 6 : 4 |
| Example 9 | - | 0.5 | 20.0 | 20.0 | | | 5 : 5 |
| Example 10 | - | 0.5 | 16.0 | 24.0 | | | 4 : 6 |

- Kollicoat® IR (BASF)
- POLYVINYL ALCOHOL (KURARAY CO.LTD.)
- HYPROMELLOSE 2910 P603 (SHIN ETSU CHEMICA CO. LTD.)
- POVIDONE K-30 (BASF)

### Examples 11 and 12: Preparation of composite preparations including tamsulosin OD tablet core coated with 5-α-reductase inhibitor-containing film coating layer

Coating solutions were prepared by mixing coating materials having the different compositions (Examples 11 and 12) presented in Table 2 with an ethanol-water mixture solution (ethanol: water = 1:1 (v/v)), and then coated on tamsulosin hydrochloride-containing Tamsulosin® OD tablets (available from Hanmi Pharmaceutical Co., Ltd., Republic of Korea) by using a pan coater (SFC-30, available from Sejong Co., Ltd.). The prepared composite tablets were dried at about 35°C for about 30 minutes, thereby preparing composite preparations including a Tamsulosin® OD tablet core coated with a 5-α-reductase inhibitor-containing film coating layer.

**[Table 2]**

| | MAIN INGREDIENT (mg) | | COATING MATERIAL (mg) | | RATIO OF KOLLICOAT® TO POLYVINYL ALCOHOL |
|---|---|---|---|---|---|
| | FINASTERIDE | DUTASTERIDE | KOLLICOAT® IR | POLYVINYL ALCOHOL | |
| EXAMPLE 11 | 5 | - | 24.0 | 16.0 | 6 : 4 |
| EXAMPLE 12 | - | 0.5 | 24.0 | 16.0 | 6 : 4 |

### Test Example 1: Defect test with respect to different coating materials

A defect test of the composite preparations of Comparative Examples 1 to 8 was performed to evaluate film tensile strengths and defect rates of the composite preparations.

Each composite preparation was packaged using a PTP packing machine (Lab-Blister machine, OMAR FANTASY PLUS), wherein an aluminum mold containing each composite preparation was sealed with an aluminum film. Ten ordinary people were randomly chosen and asked to break 100 PTP packages so as to unpack each composite formulation, wherein, as illustrated in FIG. 2, a composite preparation was counted as a defective product when the film coating layer thereof was separated from its core or was broken, or as an acceptable product when the film coating layer remained the same as before the packaging, and a percentage of the defective products (hereinafter, referred as a defect rate) was calculated. Photographic images of examples of composite preparations counted as an acceptable product (a) and a defective product (b) are shown in FIG. 2.

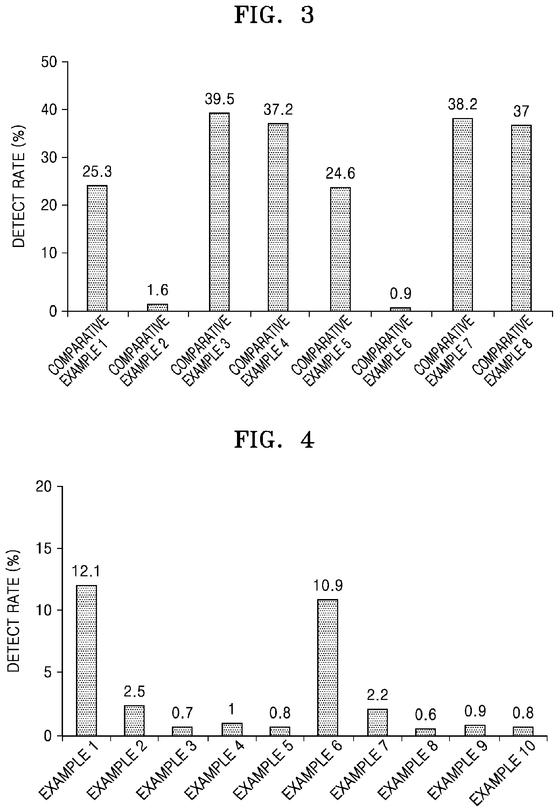

The resulting defect rates of the composite preparations are shown in Table 3 and FIG. 3.

**[Table 3]**

| | DETECT RATE (%) | COATING MATERIAL |
|---|---|---|
| COMPARATIVE EXAMPLE 1 | 25.3 | KOLLICOAT® IR |
| COMPARATIVE EXAMPLE 2 | 1 .6 | POLYVINYL ALCOHOL |
| COMPARATIVE EXAMPLE 3 | 39.5 | POVIDONE |
| COMPARATIVE EXAMPLE 4 | 37.2 | HYPROMELLOSE |
| COMPARATIVE EXAMPLE 5 | 24.6 | KOLLICOAT® IR |
| COMPARATIVE EXAMPLE 6 | 0.9 | POLYVINYL ALCOHOL |
| COMPARATIVE EXAMPLE 7 | 38. 2 | POVIDONE |
| COMPARATIVE EXAMPLE 8 | 37.0 | HYPROMELLOSE |

Referring to Table 3 and FIG. 3, the composite preparations of Comparative Examples 1, 3, 4, 5, 7, and 8 using only Kollicoat® IR, povidone, or hypromellose alone as a coating material were found to have damage of the film coating layer with a high defect rate of about 20 % to about 40 %, while the composite preparations of Comparative Example 2 and Comparative Example 6 using polyvinyl alcohol alone as a coating material were found to have a low defect rate of about 2% or less.

### Test Example 2: Defect test of composite preparations including a combination of polyvinyl alcohol-polyethylene glycol graft copolymer and polyvinyl alcohol as coating material

A defect test of the composite preparations of Examples 1 to 10 was performed in the same manner and under the same conditions as those in Test Example 1. The results are shown in Table 4 and FIG. 4.

**[Table 4]**

| | DETECT RATE (%) | RATIO OF KOLLICOAT® IR TO POLWINYL ALCOHOL |
|---|---|---|
| EXAMPLE 1 | 12.1 | 8 : 2 |
| EXAMPLE 2 | 2.5 | 7 : 3 |
| EXAMPLE 3 | 0.7 | 6 : 4 |
| EXAMPLE 4 | 1.0 | 5 : 5 |
| EXAMPLE 5 | 0.8 | 4 : 6 |
| EXAMPLE 6 | 10.9 | 8 : 2 |
| EXAMPLE 7 | 2.2 | 7 : 3 |
| EXAMPLE 8 | 0. 6 | 6 : 4 |
| EXAMPLE 9 | 0.9 | 5 : 5 |
| EXAMPLE 10 | 0.8 | 4 : 6 |

Referring to Tables 3 and 4, the composite preparations of Examples 1 to 10 using a combination of Kollicoat® IR and polyvinyl alcohol as a coating material were found to have a remarkably reduced defect rate, as compared with the composite preparations of Comparative Examples 1 and 5 using Kollicoat® IR alone as a coating material.

In particular, the composite preparations of Examples 2 to 5 and Examples 7 to 10 using Kollicoat® IR and polyvinyl alcohol in a ratio of about 7:3 to about 4:6 were found to have a lowered defect rate, as compared with the composite preparations of Examples 1 and 6 using Kollicoat® IR and polyvinyl alcohol in a ratio of about 8:2.

### Test Example 3: Defect test under stress conditions with respect to different coating materials

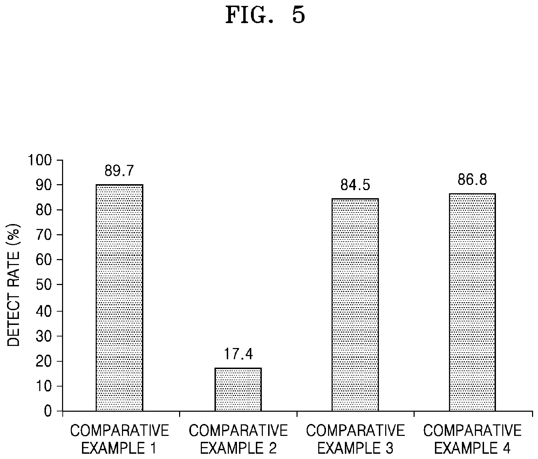

A defect test of the composite preparations of Comparative Examples 1 to 4 was performed in the same manner as in Test Example 1 after storage in a thermostatic chamber at about 60°C and at a relative humidity (RH) of 0% for one week, to evaluate stability of each composite preparation with respect to storage time. The test results are shown in Table 5 and FIG. 5.

**[Table 5]**

| | DETECT RATE (%) | COATING MATERIAL |
|---|---|---|
| COMPARATIVE EXAMPLE 1 | 89.7 % | KOLLICOAT® IR |
| COMPARATIVE EXAMPLE 2 | 17.4 % | POLYVINYL ALCOHOL |
| COMPARATIVE EXAMPLE 3 | 84.5 % | POVIDONE |
| COMPARATIVE EXAMPLE 4 | 86.8 % | HYPROMELLOSE |

Referring to Table 5 and FIG. 5, the composite preparations of Comparative Examples 1, 3, and 4 using Kollicoat® IR, povidone, or hypromellose alone as a coating material were all found to have a high defect rate of about 80% or greater. However, the composite preparation of Comparative Example 2 using polyvinyl alcohol as a coating material was found to have a defect rate of about 17.4%, exhibiting improved shape and stability, as compared with the composite preparations of Comparative Example 1, Comparative Example 3, and Comparative Example 4.

### Test Example 4: Dissolution test with respect to different coating materials

A dissolution test was performed as follows to evaluate a dissolution rate of finasteride in each of the composite preparations of Comparative Examples 1 to 4.

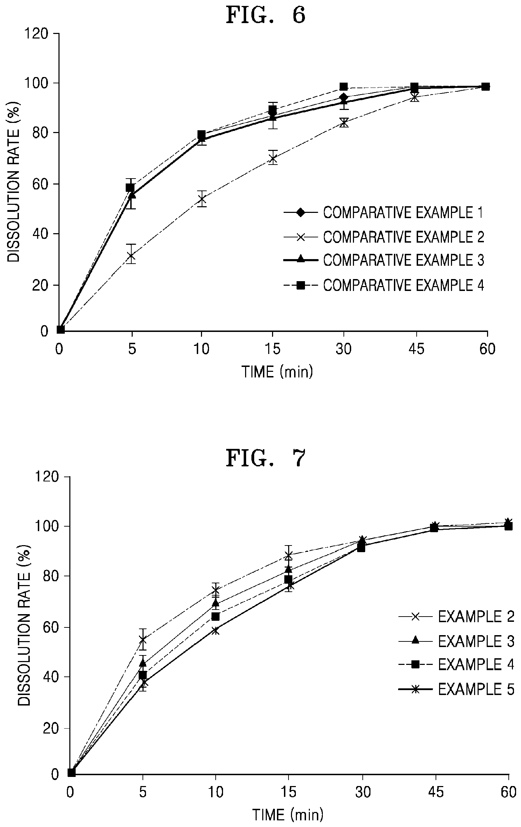

The dissolution test was performed using Dissolution Method II (Paddle method) of the General Tests of the Korean Pharmacopoeia (10th edition). In accordance with the Korean Pharmacopoeia (10th edition), 900 mL of distilled water (DW) was used as a test solution, and the dissolution test was performed at a speed of about 50 rpm according to an general operation method for release formulations. The test solution was taken at 0 minutes, 5 minutes, 10 minutes, 15 minutes, 30 minutes, 45 minutes, and 60 minutes after beginning the test, and analyzed by liquid chromatography according to the General Tests of the Korean Pharmacopoeia (10th edition), thereby obtaining a dissolution rate at each point of time through comparison with a previously prepared standard solution. The results are shown in Table 6 and FIG. 6.

**[Table 6]**

| | COMPARATIVE EXAMPLE 1 | | COMPARATIVE EXAMPLE 2 | | COMPARATIVE EXAMPLE 3 | | COMPARATIVE EXAMPLE 4 | |
|---|---|---|---|---|---|---|---|---|
| TIME (MIN) | AVERAGE | SD | AVERAGE | SD | AVERAGE | SD | AVERAGE | SD |
| 0 | 0.0 | 0.0 | 0.0 | 0.0 | 0.0 | 0.0 | 0.0 | 0.0 |
| 5 | 56.7 | 3.4 | 30.8 | 4.1 | 54.8 | 5.4 | 58.0 | 3.3 |
| 10 | 79.6 | 1.5 | 53.8 | 3.3 | 77.9 | 2.1 | 79.8 | 1.9 |
| 15 | 88.0 | 2.2 | 70.3 | 2.9 | 86.6 | 4.1 | 89.8 | 2.7 |
| 30 | 94.8 | 1.0 | 84.2 | 2.0 | 93.5 | 3.5 | 98.8 | 0.9 |
| 45 | 99.4 | 1.7 | 95.4 | 2.2 | 98.2 | 0.5 | 99.0 | 1.2 |
| 60 | 99.6 | 0.8 | 98.6 | 1.8 | 98.9 | 0.8 | 99.2 | 0.9 |

Referring to Table 6 and FIG. 6, the composite preparations of Comparative Examples 1, 3, and 4 using Kollicoat® IR, povidone, or hypromellose alone, were found to have a relatively high dissolution rate, as compared with the composite preparation of Comparative Example 2 using polyvinyl alcohol.

Finasteride is a drug which requires a high dissolution rate to achieve high bioavailability. Accordingly, the composite preparation of Comparative Example 2 using polyvinyl alcohol may have lower bioavailability, as compared with the composite preparations of Comparative Example 1, 3, and 4.

### Test Example 5: Dissolution test of composite preparations including a combination of polyvinyl alcohol-polyethylene glycol graft copolymer and polyvinyl alcohol as coating material

A dissolution test of the composite preparations of Examples 2 to 5 was performed under the same conditions as those in Test Example 4. The results are shown in Table 7 and FIG. 7.

**[Table 7]**

| | EXAMPLE 2 | | EXAMPLE 3 | | EXAMPLE 4 | | EXAMPLE 5 | |
|---|---|---|---|---|---|---|---|---|
| TIME (MIN) | AVERAGE | SD | AVERAGE | SD | AVERAGE | SD | AVERAGE | SD |
| 0 | 0.0 | 0.0 | 0.0 | 0.0 | 0.0 | 0.0 | 0.0 | 0.0 |
| 5 | 54.3 | 4.1 | 45.0 | 3.3 | 40.2 | 5.3 | 37.1 | 3.5 |
| 10 | 73.6 | 2.9 | 69.2 | 2.5 | 63.9 | 2.2 | 58.3 | 1.3 |
| 15 | 87.9 | 4.0 | 83.1 | 3.5 | 78.3 | 2.9 | 16.1 | 2.5 |
| 30 | 93.8 | 1.1 | 94.1 | 1.2 | 92.1 | 1.5 | 92.2 | 1.8 |
| 45 | 99.4 | 0.6 | 100.2 | 0.9 | 98.9 | 1.6 | 98.2 | 2.4 |
| 60 | 99.6 | 0.9 | 101.2 | 0.7 | 100.2 | 2.0 | 99.7 | 0.5 |

Referring to Table 7 and FIG. 7, the composite preparations of Examples 2 to 5 using Kollicoat® IR and polyvinyl alcohol in a weight ratio of about 7:3 to about 4:6 were found to have a dissolution rate of about 75% or greater in 15 minutes.

### Test Example 6: Defect test with respect to core type of composite preparation including a combination of polyvinyl alcohol-polyethylene glycol graft copolymer and polyvinyl alcohol as coating material

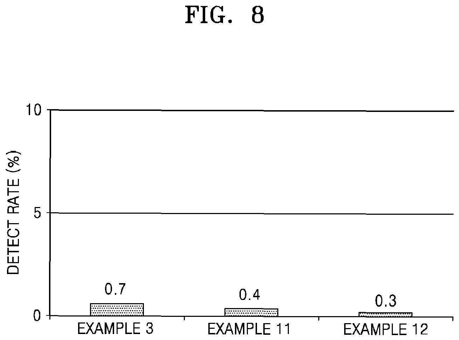

A defect test of the composite preparations of Examples 3, 11, and 12 was performed in the same manner and under the same conditions as those in Test Example 1. The results are shown in Table 8 and FIG. 8.

**[Table 8]**

| | DETECT RATE (%) | RATIO OF KOLLICOAT® IR TO POLYVINYL ALCOHOL |
|---|---|---|
| EXAMPLE 3 | 0.7 % | 6 : 4 |
| EXAMPLE 11 | 0.4 % | 6 : 4 |
| EXAMPLE 12 | 0.3 % | 6 : 4 |

As a result of the defect test, the composite preparations of Examples 11 and 12 using a tamsulosin tablet core and the same coating material as the composition preparation of Example 3 (using a tamsulosin capsule core), which was found to have an improved coating shape and stability with a high dissolution rate, were also found to have a low defect rate of about 1% or less. Therefore, the second active ingredient-containing film coating layer of a composite preparation according to any of the embodiments was found to be applicable to various core types, not only to hard capsule cores.

## Claims

1. A composite preparation comprising:
a core containing a first active ingredient; and
a film coating layer containing a second active ingredient,
wherein the film coating layer comprises a polyvinyl alcohol-polyethylene glycol graft copolymer and polyvinyl alcohol,
wherein a weight ratio of the polyvinyl alcohol-polyethylene glycol graft copolymer to the polyvinyl alcohol is 7:3 to 4:6, and
wherein the second active ingredient comprises a 5-α-reductase inhibitor selected from the group consisting of finasteride, dutasteride and any combination thereof.

2. The composite preparation of claim 1, wherein the core is a tablet, a hard capsule, or a soft capsule.

3. The composite preparation of claim 1, wherein the polyvinyl alcohol-polyethylene glycol graft copolymer consists of 65% to 85% of a polyvinyl alcohol unit and 15% to 35% of a polyethylene glycol unit, contains 0.01 wt% to 0.5 wt% of colloid silica, and has a weight average molecular weight of 35,000 Daltons to 55,000 Daltons.

4. The composite preparation of claim 1, wherein the polyvinyl alcohol has a molecular weight of 20,000 Daltons to 200,000 Daltons.

5. The composite preparation of claim 1, wherein the polyvinyl alcohol-polyethylene glycol graft copolymer consists of 65% to 85% of a polyvinyl alcohol unit and 15% to 35% of a polyethylene glycol unit, contains 0.01 wt% to 0.5 wt% of colloid silica, and has a weight average molecular weight of 35,000 to 55,000 Daltons;
the polyvinyl alcohol has a molecular weight of 20,000 Daltons to 200,000 Daltons.

6. The composite preparation of claim 1, wherein the first active ingredient comprises tamsulosin or a pharmaceutically acceptable salt thereof.

7. The composite preparation of claim 1, wherein the first active ingredient comprises 0.1 mg to 0.6 mg of tamsulosin or a pharmaceutically acceptable salt thereof, expressed in terms of the amount of free base, and the second active ingredient comprises 1 mg to 10 mg of finasteride or 0.2 mg to 0.8 mg of dutasteride.

8. The composite preparation of claim 1, wherein the first active ingredient comprises a phosphodiesterase-5 inhibitor.

9. The composite preparation of claim 8, wherein the phosphodiesterase-5 inhibitor is tadalafil or a pharmaceutically acceptable salt thereof.

10. The composite preparation of claim 9, wherein the first active ingredient comprises 5 mg to 20 mg of tadalafil or a pharmaceutically acceptable salt thereof, expressed in terms of the amount of free base, and the second active ingredient comprises 1 mg to 10 mg of finasteride or 0.2 mg to 0.8 mg of dutasteride.

11. The composite preparation of any one of claims 6 to 10, wherein the composite preparation has a 5-α-reductase inhibitor dissolution rate of 75% or greater in 15 minutes, as evaluated using a Dissolution Test according to the General Tests of the *Korean Pharmacopoeia.*

12. A method of preparing a composite preparation according to any one of claims 1 to 10, the method comprising:
preparing the core containing the first active ingredient;
preparing a second active ingredient-containing coating solution in which the second active ingredient, a polyvinyl alcohol-polyethylene glycol graft copolymer, and polyvinyl alcohol are dissolved in a solvent; and
coating the core with the second active ingredient-containing coating solution,
wherein a weight ratio of the polyvinyl alcohol-polyethylene glycol graft copolymer to the polyvinyl alcohol is 7:3 to 4:6, and
wherein the second active ingredient comprises a 5-α-reductase inhibitor selected from the group consisting of finasteride, dutasteride and any combination thereof.

13. The method of claim 12, wherein the solvent is water, ethanol, methanol, chloroform, dimethyl sulfoxide (DMSO), or a mixed solution thereof.

## Patentansprüche

1. Zusammengesetzte Zubereitung umfassend:
einen Kern, der einen ersten Wirkstoff enthält; und
eine Filmbeschichtungsschicht, die einen zweiten Wirkstoff enthält,
wobei die Filmbeschichtungsschicht ein Polyvinylalkohol-Polyethylenglykol-Pfropfcopolymer und Polyvinylalkohol umfasst,
wobei ein Gewichtsverhältnis des Polyvinylalkohol-Polyethylenglykol-Pfropfcopolymers zu dem Polyvinylalkohol 7:3 bis 4:6 beträgt und
wobei der zweite Wirkstoff einen 5-α-Reduktaseinhibitor umfasst ausgewählt aus der Gruppe bestehend aus Finasterid, Dutasterid und irgendeiner Kombination davon.

2. Zusammengesetzte Zubereitung nach Anspruch 1, wobei der Kern eine Tablette, eine Hartkapsel oder eine Weichkapsel ist.

3. Zusammengesetzte Zubereitung nach Anspruch 1, wobei das Polyvinylalkohol-Polyethylenglykol-Pfropfcopolymer zu 65 % bis 85 % aus einer Polyvinylalkoholeinheit und 15 % bis 35 % aus einer Polyethylenglykoleinheit besteht, 0,01 Gew.-% bis 0,5 Gew.-% kolloidales Siliciumdioxid enthält und ein gewichtsdurchschnittliches Molekulargewicht von 35.000 Dalton bis 55.000 Dalton aufweist.

4. Zusammengesetzte Zubereitung nach Anspruch 1, wobei der Polyvinylalkohol ein Molekulargewicht von 20.000 Dalton bis 200.000 Dalton aufweist.

5. Zusammengesetzte Zubereitung nach Anspruch 1, wobei das Polyvinylalkohol-Polyethylenglykol-Pfropfcopolymer zu 65 % bis 85 % aus einer Polyvinylalkoholeinheit und 15 % bis 35 % aus einer Polyethylenglykoleinheit besteht, 0,01 Gew.-% bis 0,5 Gew.-% kolloidales Siliciumdioxid enthält und ein gewichtsdurchschnittliches Molekulargewicht von 35.000 Dalton bis 55.000 Dalton aufweist;
der Polyvinylalkohol ein Molekulargewicht von 20.000 bis 200.000 Dalton aufweist

6. Zusammengesetzte Zubereitung nach Anspruch 1, wobei der erste Wirkstoff Tamsulosin oder ein pharmazeutisch akzeptables Salz davon umfasst.

7. Zusammengesetzte Zubereitung nach Anspruch 1, wobei der erste Wirkstoff 0,1 mg bis 0,6 mg Tamsulosin oder ein pharmazeutisch akzeptables Salz davon, als Menge freier Base ausgedrückt, umfasst und der zweite Wirkstoff 1 mg bis 10 mg Finasterid oder 0,2 mg bis 0,8 mg Dutasterid umfasst.

8. Zusammengesetzte Zubereitung nach Anspruch 1, wobei der erste Wirkstoff Phosphodiesterase-5-Inhibitor umfasst.

9. Zusammengesetzte Zubereitung nach Anspruch 8, wobei der Phosphodiesterase-5-Inhibitor Tadalafil oder ein pharmazeutisch akzeptables Salz davon ist.

10. Zusammengesetzte Zubereitung nach Anspruch 9, wobei der erste Wirkstoff 5 mg bis 20 mg Tadalafil oder ein pharmazeutisch akzeptables Salz davon, als Menge freier Base ausgedrückt, umfasst und der zweite Wirkstoff 1 mg bis 10 mg Finasterid oder 0,2 mg bis 0,8 mg Dutasterid umfasst.

11. Zusammengesetzte Zubereitung nach irgendeinem der Ansprüche 6 bis 10, wobei die zusammengesetzte Zubereitung eine 5-α- Reduktaseinhibitor-Lösungsrate von 75 % oder mehr in 15 Minuten, wie unter Anwendung eines Lösungstests den allgemeinen Tests des *koreanischen Arzneibuchs* entsprechend beurteilt, aufweist.

12. Verfahren für die Herstellung einer zusammengesetzten Zubereitung nach irgendeinem der Ansprüche 1 bis 10, wobei das Verfahren Folgendes umfasst:
Herstellen des Kerns, der den ersten Wirkstoff enthält;
Herstellen einer einen zweiten Wirkstoff enthaltenden Beschichtungslösung, wobei der zweite Wirkstoff, ein Polyvinylalkohol-Polyethylenglykol-Pfropfcopolymer und Polyvinylalkohol in einem Lösungsmittel gelöst sind; und
Beschichten des Kerns mit den zweiten Wirkstoff enthaltender Beschichtungslösung,
wobei ein Gewichtsverhältnis des Polyvinylalkohol-Polyethylenglykol-Pfropfcopolymers zu dem Polyvinylalkohol 7:3 bis 4:6 beträgt, und
wobei der zweite Wirkstoff einen 5-α-Reduktaseinhibitor umfasst ausgewählt aus der Gruppe bestehend aus Finasterid, Dutasterid und irgendeiner Kombination davon.

13. Verfahren nach Anspruch 12, wobei das Lösungsmittel Wasser, Ethanol, Methanol, Chloroform, Dimethylsulfoxid (DMSO) oder eine gemischte Lösung davon ist.

## Revendications

1. Préparation composite comprenant:
un cœur contenant un premier ingrédient actif; et
une couche de revêtement de film contenant un second ingrédient actif,
la couche de revêtement de film comprenant un copolymère greffé de polyalcool de vinyle-polyéthylène glycol et du polyalcool de vinyle,
un rapport en poids du copolymère greffé de polyalcool de vinyle-polyéthylène glycol au polyalcool de vinyle étant de 7:3 à 4:6, et
le second ingrédient actif comprenant un inhibiteur de 5-α-réductase sélectionné dans le groupe constitué du finastéride, du dutastéride et de n'importe quelle combinaison de ceux-ci.

2. Préparation composite selon la revendication 1, le cœur étant un comprimé, une capsule dure, ou une capsule molle.

3. Préparation composite selon la revendication 1, le copolymère greffé de polyalcool de vinyle-polyéthylène glycol étant constitué de 65 % à 85 % d'un motif polyalcool de vinyle et de 15 % à 35 % d'un motif polyéthylène glycol, contient de 0,01 % en pds à 0,5 % en pds de silice colloïdale, et présente un poids moléculaire moyen en poids de 35 000 Daltons à 55 000 Daltons.

4. Préparation composite selon la revendication 1, le polyalcool de vinyle ayant un poids moléculaire de 20 000 Daltons à 200 000 Daltons.

5. Préparation composite selon la revendication 1, le copolymère greffé de polyalcool de vinyle-polyéthylène glycol étant constitué de 65 % à 85 % d'un motif polyalcool de vinyle et de 15 % à 35 % d'un motif polyéthylène glycol, contient de 0,01 % en pds à 0,5 % en pds de silice colloïdale, et présente un poids moléculaire moyen en poids de 35 000 à 55 000 Daltons;
le polyalcool de vinyle présente un poids moléculaire de 20 000 Daltons à 200 000 Daltons.

6. Préparation composite selon la revendication 1, le premier ingrédient actif comprenant de la tamsulosine ou son sel pharmaceutiquement acceptable.

7. Préparation composite selon la revendication 1, le premier ingrédient actif comprenant de 0,1 mg à 0,6 mg de tamsulosine ou son sel pharmaceutiquement acceptable, exprimé en termes de la quantité de base libre, et le second ingrédient actif comprenant de 1 mg à 10 mg de finastéride ou de 0,2 mg à 0,8 mg de dutastéride.

8. Préparation composite selon la revendication 1, le premier ingrédient actif comprenant un inhibiteur de phosphodiestérase-5.

9. Préparation composite selon la revendication 8, l'inhibiteur de phosphodiestérase-5 étant le tadalafil ou son sel pharmaceutiquement acceptable.

10. Préparation composite selon la revendication 9, le premier ingrédient actif comprenant de 5 mg à 20 mg de tadalafil ou son sel pharmaceutiquement acceptable, exprimé en termes de la quantité de base libre, et le second ingrédient actif comprenant de 1 mg à 10 mg de finastéride ou de 0,2 mg à 0,8 mg de dutastéride.

11. Préparation composite selon l'une quelconque des revendications 6 à 10, la préparation composite ayant un taux de dissolution d'inhibiteur de 5-α-réductase de 75 % ou plus en 15 minutes, tel qu'évalué en utilisant un test de dissolution selon les tests généraux de la *Pharmacopée coréenne.*

12. Procédé de préparation d'une préparation composite selon l'une quelconque des revendications 1 à 10, le procédé comprenant:
la préparation du cœur contenant le premier ingrédient actif;
la préparation d'une solution de revêtement contenant le second ingrédient actif dans laquelle le second ingrédient actif, un copolymère greffé de polyalcool de vinyle-polyéthylène glycol, et du polyalcool de vinyle sont dissous dans un solvant; et
le revêtement du cœur avec la solution de revêtement contenant le second ingrédient actif, un rapport en poids du copolymère greffé de polyalcool de vinyle-polyéthylène glycol au polyalcool de vinyle étant de 7:3 à 4:6, et
le second ingrédient actif comprenant un inhibiteur de 5-α-réductase sélectionné dans le groupe constitué du finastéride, du dutastéride et de n'importe quelle combinaison de ceux-ci.

13. Procédé selon la revendication 12, le solvant étant l'eau, l'éthanol, le méthanol, le chloroforme, le sulfoxyde de diméthyle (DMSO), ou une solution mixte de ceux-ci.
